# EUROPEAN PATENT SPECIFICATION

(11) **EP 2 838 346 B1**
(45) Date of publication and mention of the grant of the patent: **14.10.2026**
(21) Application number: 13719448.6
(22) Date of filing: 18.04.2013
(51) Int. Cl.: A01H 5/10, A01H 5/12, A01H 1/04, C12N 15/29, C12Q 1/68

(54) **THRIPS RESISTANT CABBAGE**
GEGEN FRANSENFLÜGLER RESISTENTER KOHL
CHOU RÉSISTANT AU THRIPS

(30) Priority: 19.04.2012 EP 12164775
(43) Date of publication of application: 25.02.2015
(73) Proprietor: Rijk Zwaan Zaadteelt en Zaadhandel B.V., 2678 KX De Lier (NL)
(72) Inventor: LÖPTIEN, Harm, 25709 Marne (DE)
(74) Representative: Arnold & Siedsma
(86) International application number: PCT/EP2013/058090
(87) International publication number: WO 2013/156566

(56) References cited:
- GRIFFITHS, P.D.: "Breeding and characterization of Thrips Resistance in Cabbage", REPORT TO THE NY IPM PROGRAM, December 2008 (2008-12-01), pages 1 - 7, XP002682968
- FAIL, J., ET AL.: "Developing methods for testing the resistance of white cabbage against Thrips tabaci", July 2001 (2001-07-01), pages 229 - 237, XP002682969, Retrieved from the Internet <URL:http://www.ento.csiro.au/thysanoptera/Symposium/Section7/36-Fail-Penzes.pdf> [retrieved on 20120906]
- ROELAND E VOORRIPS ET AL: "Plant traits associated with resistance to Thrips tabaci in cabbage (Brassica oleracea var capitata)", EUPHYTICA, KLUWER ACADEMIC PUBLISHERS, DO, vol. 163, no. 3, 30 May 2008 (2008-05-30), pages 409 - 415, XP019603921, ISSN: 1573-5060
- TRDAN S ET AL: "Feeding damage by onion thrips, Thrips tabaci Lindeman (Thysanoptera : Thripidae), on early white cabbage grown under insecticide-free conditions", AFRICAN ENTOMOLOGY, vol. 13, no. 1, March 2005 (2005-03-01), pages 85 - 95, XP002682970, ISSN: 1021-3589

## Description

### Field of the invention

The present invention relates to *Brassica* plants, in particular *Brassica oleracea* plants resistant to *Thrips tabaci,* to seeds and progeny from the seeds and plants.

### Background of the invention

The present invention relates to *Brassica* plants, in particular *Brassica oleracea* plants which are resistant to *Thrips tabaci* and herein display agronomically desirable traits.

*Thrips tabaci* (also known as Onion thrips, or simply thrips) is a highly polyphagous insect and considered as an economically harmful pest for many cultivated crops especially for crops from the Liliaceae, Solanaceae, and Brassicaceae plant families, such as onion, leek, tomato, cabbage, but also in crops from outside these families, such as e.g. cucumber, melon and snap beans (Toda & Murai, 2007, Applied Entomology and Zoology 42: 309-316; Trdan et al., 2005, African Entomology 13: 85-95).

Thrips harm the plant directly by feeding on the plant tissue and indirectly by being a vector for lethal plant viruses such as tomato spotted wild virus (Toda & Murai, 2007, Applied Entomology and Zoology 42: 309-316). Thrips occur worldwide and due to climate change and trade the frequency of thrips infestation is increasing (Trdan et al., 2005; African Entomology 13: 85-95).

Cabbage plants (*Brassica oleracea* var. *capitata* L.) belong to the plant family Brassicaceae. The family has a cosmopolitan distribution and consists of approximately 3500 to 4000 species. The family and especially the genus *Brassica* contains many agronomically important crops such as broccoli, Brussels sprouts, cauliflower, Chinese cabbage, curly cabbage, kale, kohlrabi, mustard, oxheart cabbage, radish, rapeseed, red cabbage, Savoy cabbage, turnip, and white cabbage.

In tissue of *Brassica oleracea* var. *capitata* plants affected by thrips callus growths will form. Over time these callus growths become brown making it necessary to remove several layers of leaves from the cabbage head before marketing. Even in storage the development of symptoms caused by thrips may increase. The reduction in head size and weight ultimately leads to a loss in yield. For *Brassica oleracea* var. *capitata* it is estimated that 75% of its total acreage suffers from thrips.

Controlling thrips in cabbage by means of applying insecticide is considered environmentally unfriendly and ineffective, because the closed leaves of the cabbage heads provide protection to the thrips, and because thrips are hard to detect, sometimes even only at harvest stage. Thrips infestation in cabbages may occur from the second exterior leaf up to the fifteenth exterior leaf, while insecticides are usually only effective up to the sixth exterior leaf of the head (Trdan et al., 2005; African Entomology 13: 85-95). Therefore, there is an urgent need for cabbage plants which are resistant against this pest.

Although no highly thrips resistant *Brassica oleraceae* varieties are known, huge differences in susceptibility exist among varieties. Certain varieties show an intermediate level of resistance, but no varieties are known that are highly resistant against *Thrips tabaci.* The genetic background of this intermediate resistance against thrips is still poorly understood and it is thought that this is a polygenic trait, inherited as a gene complex in which many genes are involved (Voorrips et al., 2008; Euphytica 163: 409-415).

Because in the present state of the art no highly thrips resistant cabbage varieties are known, it is the object of the present invention to provide a *Brassica* plants, in particular *Brassica oleracea* plant with a high resistance to *Thrips tabaci.*

In the research that led to the present invention novel *Brassica oleracea* plants were developed that are highly resistant against thrips.

The said resistance of the invention is controlled by a genetic determinant, the inheritance of which is consistent with that of a monogenic additive trait. 'Additive trait' in this case means that the fully achievable resistance is only observable in plants comprising the genetic determinant in homozygous state, however plants comprising the genetic determinant in heterozygous state will show an intermediate level of resistance.

Since the inheritance of the resistance is comparable to that of a monogenic trait, it has a second advantage over the prior art, because the resistance level is not only higher but also easier to incorporate in new Brassica varieties as compared to the complex polygenic traits from the prior art which led to plants with only an intermediate resistance against thrips.

The present invention thus relates to a Brassica plant, in particular a *Brassica oleracea* plant, that is highly resistant to *Thrips tabaci,* wherein highly resistant is characterized by the *Brassica* plant showing no symptoms and no damage, wherein the high resistance is conferred by a QTL, which is located on chromosome 2 and linked to marker BO00200 (SEQ ID NO: 1) and/or marker BO00277 (SEQ ID NO: 2) and/or marker BO00602 (SEQ ID NO: 5), and which plant is obtainable by crossing a highly resistant plant of which representative seed was deposited under NCIMB number 41760 with another cabbage plant to produce an F1 and subsequently selfing the F1 to obtain an F2 and selecting a plant therefrom that shows high resistance to *Thrips tabaci.*

Disclosed herein is a Brassica plant, in particular *a Brassica oleracea* plant, comprising a genetic determinant, which when homozygously present confers high resistance against *Thrips tabaci,* and which is as found in plants grown from seeds of which a representative sample is deposited with the NCIMB under NCIMB accession number 41760

Also disclosed herein is a Brassica plant, in particular a *Brassica oleracea* plant, comprising the genetic determinant heterozygously, and thus showing intermediate resistance against *Thrips tabaci,* and which genetic determinant is obtainable from plants grown from seeds of which a representative sample is deposited with the NCIMB under NCIMB accession number 41760.

Further disclosed herein is a Brassica plant comprising the genetic determinant homozygously, and therefore is highly resistant against *Thrips tabaci.*

Furthermore, it was found during the research leading to the present invention that the genetic determinant conferring resistance to *Thrips tabaci,* is located on chromosome 2 and linked to marker BO00200 (SEQ ID NO: 1) and/or marker BO00277 (SEQ ID NO: 2) and/or marker BO00602 (SEQ ID NO: 5).

More in particular, in the deposit NCIMB 41760 the genetic determinant conferring resistance to *Thrips tabaci,* is located on chromosome 2 between marker BO00458 (SEQ ID NO: 7) and marker BO01225 (SEQ ID NO: 6) and linked to marker BO00200 (SEQ ID NO: 1) and/or marker BO00277 (SEQ ID NO: 2) and/or marker BO00602 (SEQ ID NO: 5).

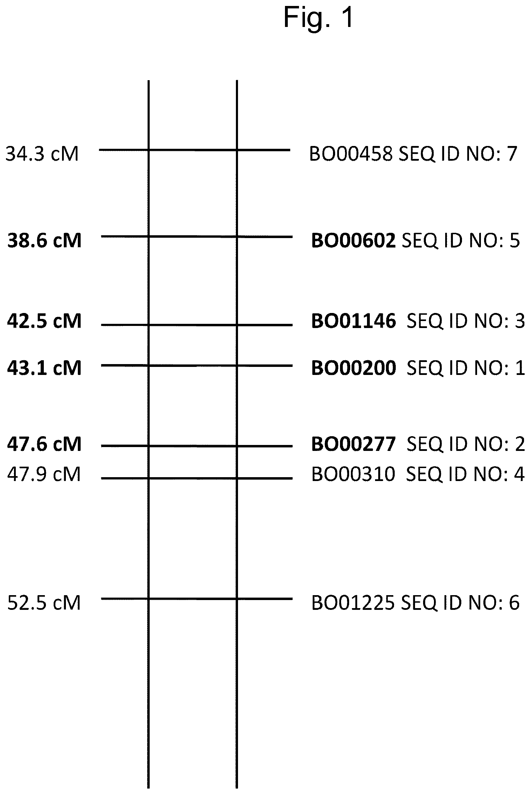

Most in particular the genetic determinant conferring resistance to *Thrips tabaci,* is located on chromosome 2 between marker BO01146 (SEQ ID NO: 3) and marker BO00310 (SEQ ID NO: 4) and linked to marker BO00200 (SEQ ID NO: 1) and/or marker BO00277 (SEQ ID NO: 2) in seeds deposited under NCIMB accession number 41760, see also Fig. 1.

Alternatively the genetic determinant conferring resistance to *Thrips tabaci,* is located on chromosome 2 between markers BO00310 (SEQ ID NO: 4) and BO00458 (SEQ ID NO: 7) and linked to BO00200 (SEQ ID NO: 1) and/or marker BO00277 (SEQ ID NO: 2) and/or marker BO01146 (SEQ ID NO: 3) and/or marker BO00602 (SEQ ID NO: 5), in particular between marker BO00458 (SEQ ID NO: 7) and marker BO01146 (SEQ ID NO: 3) and linked to marker BO00602 (SEQ ID NO: 5).

Therefore, disclosed herein is a Brassica plant resistant to *Thrips tabaci,* comprising a genetic determinant that confers resistance to *Thrips tabaci,* wherein said genetic determinant is obtainable by introgression from a plant grown from seeds of which a representative sample was deposited with the NCIMB under NCIMB accession number 41760, and wherein the said genetic determinant in the seeds of the seed deposit number NCIMB 41760 is positioned on chromosome 2 and linked to marker BO00200 (SEQ ID NO: 1), and/or BO00277 (SEQ ID NO: 2) and/or marker BO00602 (SEQ ID NO: 5).

A Brassica plant carrying the genetic determinant conferring resistance against thrips can suitably be identified among descendants from a cross between a plant susceptible for thrips, and a plant that does carry the genetic determinant in homozygous state, by growing F2 plants from seeds that are the result from the initial cross and a selfing step, and selecting plants showing the desired trait. Selecting the plants can be done phenotypically, or can be done through identification of the genetic determinant, for example by means of one or more of the markers defined herein.

In the absence of molecular markers or in the event that recombination between the molecular markers and the genetic determinant have taken place and thus are not predictive anymore, equivalence of genetic determinants can still be determined by an allelism test. To perform an allelism test, material that is homozygous for the known determinant, a tester plant, is crossed with material that is homozygous for the genetic determinant that is to be tested. This latter plant is referred to as the donor plant. The donor plant to be tested should be or should be made homozygous for the genetic determinant to be tested. The skilled person knows how to obtain a plant that is homozygous for the genetic determinant to be tested. When in the F2 of the cross between a donor plant and a tester plant no segregation for the phenotype related to the genetic determinant is observed, the genetic determinants of the donor plant and the tester plant have been proven to be equivalent or the same.

Furthermore, disclosed herein is a Brassica plant that comprises a genetic determinant conferring resistance to *Thrips tabaci,* wherein plants of first generation progeny (F1) of a cross of the said plant with a tester plant, that comprises the said genetic determinant and of which representative seed was deposited with the NCIMB under accession number NCIMB 41760, or a progeny plant thereof that comprises the said genetic determinant, or a plant derived therefrom and comprising the said genetic determinant, show a 1:0 segregation for the resistance against *Thrips tabaci.* In both the tester plant and the plant disclosed herein the genetic determinant is present in homozygous form. Plants of the second and further generations, if obtained by selfing also show a 1:0 segregation for the said resistance pattern. The tester plant can be a plant of which representative seed was deposited with the NCIMB under accession number NCIMB 41760.

The Brassica plant of the invention is preferably a *Brassica oleracea* plant, but can also be any other plant of the genus Brassica into which the skilled person can introgress the genetic determinant as disclosed herein, e.g. the following Brassica species: *Brassica oleracea, Brassica napus, Brassic campestris, Brassica cretica, Brassica rapa, Brassica juncea,* and *Brassica nigra.* The skilled person knows how to make interspecific crosses with these species, e.g. by means of embryo rescue, protoplast fusion, and other related technologies.

In another embodiment the invention relates to seeds capable of growing into a plant of the invention. Disclosed herein is that the seed may comprising the said genetic determinant conferring resistance against *Thrips tabaci. A* plant grown from the seeds is highly resistant to thrips when the genetic determinant is present in homozygous state.

According to a further aspect thereof, the invention relates to propagation material capable of growing into a plant of the invention.

In one embodiment, such propagation material is formed by seed of a Brassica plant of the invention, wherein the plant that can be grown from the seed comprises the QTL, which is located on chromosome 2 and linked to marker BO00200 (SEQ ID NO: 1) and/or marker BO00277 (SEQ ID NO: 2) and/or marker BO00602 (SEQ ID NO: 5).

In another embodiment the propagation material capable of growing into a plant of the invention is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, and cells.

In a further embodiment the invention relates to tissue culture of propagation material capable of growing into a plant of the invention.

Suitably, the plant produced from the propagation material comprises the genetic determinant as found in *Brassica* plants grown from seeds of seed lot AG6359-35/39, a representative sample of which was deposited with the NCIMB under NCIMB accession number 41760. When the genetic determinant is present in homozygous state the plant produced shows high resistance to *Thrips tabaci,* in particular the resistance as observed in *Brassica* plants grown from seeds of seed lot AG6359-35/39, a representative of which was deposited with the NCIMB under NCIMB accession number 41760. When the genetic determinant is present in heterozygous state the plant shows intermediate resistance.

The invention also relates to progeny of the plants, cells, tissues and seeds of the invention. Such progeny can in itself be plants, cells, tissues or seeds.

As used herein the word "progeny" is intended to mean the first and all further descendants from a cross with a plant of the invention that comprises the said genetic determinant. "Progeny" also encompasses plants that carry the trait of the invention and are obtained from other plants or progeny of plants of the invention by vegetative propagation or multiplication.

Therefore, in one embodiment the invention relates to progeny of a *Brassica* plant comprising the QTL, which is located on chromosome 2 and linked to marker BO00200 (SEQ ID NO: 1) and/or marker BO00277 (SEQ ID NO: 2) and/or marker BO00602 (SEQ ID NO: 5).

In a further embodiment the invention relates to progeny of *Brassica* plants of the invention that are resistant against *Thrips tabaci.*

In one aspect the invention relates to the harvested part of a *Brassica* plant comprising the QTL, which is located on chromosome 2 and linked to marker BO00200 (SEQ ID NO: 1) and/or marker BO00277 (SEQ ID NO: 2) and/or marker BO00602 (SEQ ID NO: 5).

The invention furthermore relates to a food product comprising one or more harvested parts of a Brassica plant comprising the QTL, which is located on chromosome 2 and linked to marker BO00200 (SEQ ID NO: 1) and/or marker BO00277 (SEQ ID NO: 2) and/or marker BO00602 (SEQ ID NO: 5).

The harvested part or food product can be or comprise a cabbage head, a curd, a stem, a leaf, a root, a sprout, a seed, or any other part of a *Brassica* plant. The harvested part may also be used for the production of biofuel. The food product or harvested part, may have undergone one or more processing steps. Such a processing step might comprise but is not limited to any one of the following treatments or combinations thereof: cutting, washing, cooking, steaming, baking, frying, pasteurizing, freezing, grinding, extracting oil, pickling, or fermenting. The processed form that is obtained is also part of this invention.

Disclosed herein is a nucleic acid molecule which is causative of resistance against *Thrips tabaci.* The said DNA molecule comprises a DNA sequence which is positioned on chromosome 2 between markers BO00458 (SEQ ID NO: 7) and BO01225 (SEQ ID NO: 6), more in particular between markers BO00458 (SEQ ID NO: 7) and BO00146 (SEQ ID NO:3) or between markers BO00146 (SEQ ID NO: 3) and BO00310 (SEQ ID NO: 4), or a part thereof.

Also disclosed herein use of the markers and said nucleic acid molecule to identify plants which are resistant against *Thrips tabaci,* and/or carry the genetic determinant conferring resistance to *Thrips tabaci.*

Therefore, disclosed herein is the use of marker BO00200 (SEQ ID NO: 1), or marker BO00277 (SEQ ID NO: 2), or marker BO00602 (SEQ ID NO: 5), or the said DNA molecule comprising a DNA sequence which is positioned on chromosome 2 between markers BO00602 (SEQ ID NO: 5) and BO01225 (SEQ ID NO: 6), or BO00146 (SEQ ID NO: 3) and BO00310 (SEQ ID NO: 4), or BO00458 (SEQ ID NO: 7) and BO00146 (SEQ ID NO: 3), or part thereof, to indentify plants resistant against *Thrips tabaci,* and/or carrying the genetic determinant conferring resistance to *Thrips tabaci.*

The skilled person knows how to develop new markers linked to a trait using already known markers, QTLs, alleles, genes or other DNA molecules that are associated with a certain trait.

Thus, disclosed herein is the use of markers BO00200 (SEQ ID NO: 1), BO00277 (SEQ ID NO: 2) and BO00602 (SEQ ID NO: 5), and the said DNA molecule, or part thereof, for developing other markers linked to the genetic determinant conferring thrips resistance.

In one embodiment, the genetic determinant conferring resistance to *Thrips tabaci* also confers resistance against other sap sucking insect species, wherein the sap sucking insect species is selected from, but not limited to the group consisting of *Aleyrodes proletella, Myzus persicae,* and *Brevicoryne brassicae.*

In addition, disclosed herein is a process for producing *Brassica* plants comprising a genetic determinant that confers resistance to *Thrips tabaci,* comprising the step of selecting said *Brassica* plants from a population of *Brassica* plants segregating for the said genetic determinant using marker BO00200 (SEQ ID NO: 1) and/or marker BO00277 (SEQ ID NO: 2) and/or BO00602 (SEQ ID NO: 5).

The term 'genetic determinant' as used herein encompasses one or more QTLs, genes, or alleles. These terms are used interchangeably. A genetic determinant can be identified by the use of a molecular marker. A genetic determinant can alternatively be identified by the position on a genetic map, or by indication of the location on a linkage group or chromosome. When a genetic determinant is not linked to a specific molecular marker any longer, but its position on a chromosome as defined on a genetic map is unaltered, this genetic determinant is still the same as when it was linked to the molecular marker. The genetic trait that it confers is therefore also still the same. The 'genetic trait' is the trait or characteristic that is conferred by the genetic determinant. The genetic trait can be identified phenotypically, for example by performing a bio-assay. However, also plant stages for which no phenotypic assay can be performed do carry the genetic information that leads to the genetic trait. 'Trait' or 'phenotypic trait' can be used instead of 'genetic trait'.

Also disclosed herein in are *Brassica* plants of the invention that carry the genetic determinant conferring resistance to *Thrips tabaci,* and having acquired said determinant by introduction of the genetic information that is responsible for the trait from a suitable source, either by conventional breeding, or genetic modification, in particular by cisgenesis or transgenesis. Cisgenesis is genetic modification of plants with a natural gene, coding for an (agricultural) trait, from the crop plant itself or from a sexually compatible donor plant. Transgenesis is genetic modification of a plant with a gene from a noncrossable species or a synthetic gene.

Furthermore, disclosed herein is the germplasm of plants of the invention. The germplasm is constituted by all inherited characteristics of an organism and encompasses at least the genetic determinant as disclosed herein. The germplasm can be used in a breeding program for the development of thrips resistant Brassica plants.

Further disclosed herein is a method for production of a *Brassica* plant is resistant against *Thrips tabaci,* comprising
a) crossing a plant comprising a genetic determinant that leads to resistance against *Thrips tabaci* with another plant;
b) selfing the resulting F1 for obtaining F2 plants;
c) selecting plants resistant to *Thrips tabaci* in the F2;
d) optionally performing one or more additional rounds of selfing or crossing, and subsequently selecting, for a plant comprising said resistance against *Thrips tabaci.*

It is clear that the parent that provides the trait of the invention is not necessarily a plant grown directly from the deposited seeds. The parent can also be a progeny plant from the seed or a progeny plant from seeds that are identified to have the trait of the invention by other means.

Also, disclosed herein is a method for production of *a Brassica* plant resistant against *Thrips tabaci,* comprising
a) crossing a plant comprising the genetic determinant that leads to resistance against *Thrips tabaci* with another plant;
b) optionally backcrossing the resulting F1 with the preferred parent;
c) selecting for plants resistant to *Thrips tabaci* in the F2;
d) optionally performing one or more additional rounds of selfing or crossing, and subsequently selecting, for a plant comprising the said resistance is performed.

Furthermore, disclosed herein is a method of introducing a desired trait into a *Brassica* plant resistant against *Thrips tabaci,* comprising:
a) crossing a *Brassica* plant resistant to *Thrips tabaci,* representative seed of which were deposited with the NCIMB under deposit number NCIMB 41760, with a second *Brassica* plant that comprises a desired trait to produce F1 progeny;
b) selecting an F1 progeny that comprises said resistance and the desired trait;
c) crossing the selected F1 progeny with either parent, to produce backcross progeny;
d) selecting backcross progeny comprising the desired trait and resistance against *Thrips tabaci;* and
e) optionally repeating steps c) and d) one or more times in succession to produce selected fourth or higher backcross progeny that comprises the desired trait and resistance against *Thrips tabaci.*

In one embodiment selection for plants resistant against *Thrips tabaci* is done in the F1 by using marker BO00200 (SEQ ID NO: 1) and/or marker BO00277 (SEQ ID NO: 2) and/or BO00602 (SEQ ID NO: 5). In another aspect selection for the trait of the invention is started in the F2 of a cross or alternatively of a backcross. Selection of plants in the F2 can be done phenotypically as well as by using the said markers.

In one embodiment selection for plants resistant against *Thrips tabaci* is started in the F3 or a later generation.

In one embodiment the plant comprising the genetic determinant is a plant of an inbred line, a hybrid, a doubled haploid, or of a segregating population.

Further disclosed herein is a method for the production of a *Brassica* plant resistant against *Thrips tabaci* by using a doubled haploid generation technique to generate a doubled haploid line comprising the said resistance.

Hybrid seed that can be grown into a thrips resistant plant and to a method for producing such hybrid seed comprising crossing a first parent plant with a second parent plant and harvesting the resultant hybrid seed, wherein said first parent plant and/or said second parent plant is the plant as claimed are also disclosed herein.

Further disclosed herein is a method for producing a hybrid *Brassica* plant that is resistant to thrips, comprising crossing a first parent *Brassica* plant with a second parent *Brassica* plant and harvesting the resultant hybrid seed, of which the first parent plant and/or the second parent plant is resistant against *Thrips tabaci,* and growing said hybrid seeds into thrips resistant hybrid plants.

In addition, disclosed herein is a method for the production of a *Brassica* plant resistant against *Thrips tabaci* by using a seed that comprises a genetic determinant in its genome that leads to resistance against *Thrips tabaci* for growing the said *Brassica* plant. The seeds are suitably seeds of which a representative sample was deposited with the NCIMB under deposit number NCIMB 41760.

Also disclosed herein is a method for seed production comprising growing *Brassica* plants from seeds of which a representative sample was deposited with the NCIMB under deposit number NCIMB 41760, allowing the plants to produce seeds, and harvesting those seeds. Production of the seeds is suitably done by crossing or selfing.

Further disclosed herein is a method for the production of a *Brassica* plant resistant against *Thrips tabaci* by using tissue culture.

Furthermore, disclosed herein is a method for the production of a *Brassica* plant resistant against *Thrips tabaci* by using vegetative reproduction.

Also disclosed herein is a method for the production of a *Brassica* plant resistant against *Thrips tabaci* by using a method for genetic modification to introgress the said resistance into the *Brassica* plant. Genetic modification comprises transgenic modification or transgenesis, using a gene from a non-crossable species or a synthetic gene, and cisgenic modification or cisgenesis, using a natural gene, coding for an (agricultural) trait, from the crop plant itself or from a sexually compatible donor plant.

Additionally, disclosed herein is a breeding method for the development of *Brassica* plants that are resistant against *Thrips tabaci* wherein germplasm comprising said resistance is used. Representative seed of said plant comprising the genetic determinant and being representative for the germplasm was deposited with the NCIMB under deposit number NCIMB 41760.

Further disclosed herein is a method for the production of a *Brassica* plant resistant against *Thrips tabaci* wherein progeny or propagation material of a plant comprising the genetic determinant conferring said resistance is used as a source to introgress the said resistance into another *Brassica* plant. Representative seed of said plant comprising the genetic determinant was deposited with the NCIMB under deposit number NCIMB 41760.

Also disclosed herein is a *Brassica* plant resistant to *Thrips tabaci,* which plant is obtainable by any of the methods herein described and/or familiar to the skilled person.

### DEPOSITED MATERIAL

Seeds of *Brassica oleracea* plants resistant to *Thrips tabaci* were deposited under NCIMB deposit accession number 41760 on 29 September 2010 with NCIMB Ltd. (Ferguson Building, Craibstone Estate, Bucksburn, Aberdeen, AB21 9YA). All seeds of the deposit comprise the genetic determinant homozygously. Plants grown from these seeds are thus highly resistant against *Thrips tabaci.*

The present invention will be further elucidated in the examples that follow. These examples are for illustration purpose only and are not to be construed as limiting this invention in any way.

In the Example reference is made to the following figure:
**Fig. 1****.** Schematic overview of a part of chromosome two indicating markers (in bold) linked to the genetic determinant conferring thrips resistance (SEQ ID NO 1, 2, 3 and 5) together with flanking markers (SEQ ID NO: 4, 6 and 7). On the left side the genetic distance indicated in centiMorgans. On the right side, the markers are indicated.

### SEQUENCE DATA

**Table 1**

| Sequence data of the SNP markers of **Fig. 1****.** In NCIMB deposit 41760 the markers BO00200, BO00277 and BO00602 are linked to the genetic determinant conferring resistance against Thrips. | |
|---|---|
| BO00200 SEQ ID NO: 1 | TTGTCCGGAAAATAAGCCCTTCCTTCTCCATGAGCCGCCCAAACTCCT AAAGTACTTACC[T/C]TCCATTCCTTTCAGCACTATTGACGGACAGT CCTTGATGGTCACGCTTGTGAACCTGCAG |
| BO00277 SEQ ID NO: 2 | GCTTGTCCAGCTTATGAGCATTTTGTAATACATGTTTGCTTGGATGGT CAAACCTGAAAA[A/C]ACACACAAGTTCAACAATTCTCAGAAGATGG ATAAACCTGCAG |
| BO01146 SEQ ID NO: 3 | TTAAAAACAAATNATGTTTTGTTTTACCTGCTTTTTCCTCTTCTCGCT NNTTGNCAGCTTCTTCTCTCTGTTGGCGAATCAGAGCCAAACGATCTG TACACAAACACACACAAGGATAAGATAATCAA[A/*]TAGGCTCATC TGGTAACAAAGAAAGAGAACAAGAGGTCCATTGTTTTTTACNNAANT CCTTNNTTGNTTGCTCGGTTTTNNCNTGNNNCTGCAACCTCATGTATC GCTCATGAGCTCGTTGCTTCTCTAGCTCCTCCCTGCAG |
| BO00310 SEQ ID NO: 4 | CATGTCATCTTTCGTNAGGTTTGTTATTTCTATGCCTCGTTTGAGAGC TTCCCTCAATGG[A/C]CCCATAGTTGCATCTTTCACTAGGTTCTTCA TGTCTGATCCCGAGTACCCTGCAG |
| BO00602 SEQ ID NO: 5 | TTAAATATGTGAATGCTGAAATRTTTGTTTAGCAGA[C/G]GAGACAC AGGAAGAAGATCATCTCATGTTGTTCGCTCTCTCAGCAGAGAGTTTGA AGTTGCAAGTACTCCTGCAG |
| BO01225 SEQ ID NO: 6 | CCCTGTTWAAGGAGCCTCCTTGGAGCTTGTTGATACCTCAAAGTTAGG TAGTGACAATGTGGATAATGAAAGTTTGAAGCTTT[A/C]TCAACAAT TAGCTGATAAGAGAGGTTCTTGTGAAGAGGATTTGATGAGAATCTCTA TGAAGAAACGAGGTGTAATCAGCAATGTCTCCACCTCTYTGATGGAAR ATGCTRGTTTYGATGGAATATTGGCTTCTCCTGCAG |
| BO00458 SEQ ID NO: 7 | TAAAGAACCTGATGAAGAAAGTGAAGAGCTAGGTGGAAACCTACTTGA CCTGCTCCTAAG[T/G]TTCTTCACTTCCGGATCTTCTCCTCTTTTG ATCTCACCTGCCTCACCTTTGCCTCGTTC |

### EXAMPLES

### EXAMPLE 1

Thrips resistant plants of the invention were compared with susceptible plants and plants with an intermediate resistance in an open field trial. Plants were naturally infected by using an attractant species. In this trial leek plants were used as a natural attractant for thrips. For the skilled person it is known that also other plants can be used as an attractant for *Thrips tabaci,* such as onion or shallots.

Plants from variety Rinda and Hurricane were used in this trial as susceptible control plants. Plants from varieties Langendijker Bewaar, Tacoma, and Multima were included in this trial since they are known to be intermediately resistant against *Thrips tabaci.* As highly resistant plants of the invention, plants grown from seeds of seed lot AG6359-35/39 of which a representative sample was deposited with the NCIMB under NCIMB accession number 41760, as well as plants of three lines derived from plants grown from seed of the seed deposit that were selfed, were used. These lines are AG2971, AG2973, and AG3086.

Each plant was scored for the amount of thrips infestation, based on the scale explained in **Table 2.** Symptoms were scored when the cabbage heads were mature. The skilled person is not bound to using this scale but can also use a scale with another subdivision of categories as long as the scoring will be done at the same stage, i.e. when the cabbage head is mature. This is not of influence on the final result of the screening.

**Tabel 2**

| Scoring of thrips infestation. | | |
|---|---|---|
| **Score** | **Symptoms** | |
| **1** | No symptoms / no damage | **High resistance** |
| **2** | Occasional small symptoms on outer leaves | |
| **3** | Minor damage. Max. 20% of head's surface is covered by symptoms, only outer leaves are infected. | **Intermediate resistance** |
| **4** | Medium damage. Max. 50% of the head's surface (all leaves accumulated) is covered by symptoms. | |
| | The symptoms are max. in the second and third layer. | |
| **5** | Heavy damage. More than 50% of the head's surface (all leaves accumulated) is covered by symptoms. | **Susceptible** |
| | Damage also observed in deeper leaf layers | |

| | | |
|---|---|---|
| In **Table 3** the scores of the trial are summarized. It is clear that all plants of the invention display a significantly higher resistance against thrips. | | |

In field trials with a high disease pressure, even highly resistant plants show occasional symptoms of thrips infection. This can e.g. be the case when attractant plant species are used. In order to make a fair comparison between different trials using natural infection the same comparison varieties should be used, because the relative differences in damage observed by thrips infestation will approximately be the same while the mean scores of same plants in different trials can deviate substantially.

**Table 3**

| Score of thrips resistance field trial. | | | |
|---|---|---|---|
| **Material** | **Number of plants screened** | **Mean score** | |
| AG6359-35/39 (NCIMB 41760) | 17 | 1 | Resistant |
| AG2971 | 14 | 1.1 | Resistant |
| AG2973 | 16 | 1.2 | Resistant |
| AG3086 | 4 | 1.2 | Resistant |
| Langendijker Bewaar | 22 | 3.1 | Intermediate |
| Tacoma | 6 | 3 | Intermediate |
| Multima | 6 | 3 | Intermediate |
| Hurricane | 10 | 4.4 | Susceptible |
| Rinda | 10 | 5 | Susceptible |

### EXAMPLE 2

### Transfer of thrips resistance to susceptible plants

A thrips resistant plants of the invention AG 2973-35 (see **Table 2** for resistance scores), was crossed with a susceptible plant FM 2979-01. Plants of the F1 were observed in a field trial as described in **Example 1.** No highly thrips resistant plants were observed.

From the F1 population a plant was selected which was selfed to obtain a population of F2 plants. The F2 was placed in a field trial as described in **Example 1.** Resistance scores are summarized in **Table 4.**

The segregation of the F2 population demonstrates that the inheritance of the resistance of the invention is comparable with that of a monogenic additive trait (highly resistant : intermediate resistant : susceptible = 1:2:1). Depending on the genetic background of the susceptible parent plant there can be a different distribution between intermediately resistant and susceptible plants observed in the F2.

**Table 4**

| **F2 population** | **Resistant plants** | **Intermediately resistant plants** | **Susceptible plants** | **Chi-square** |
|---|---|---|---|---|
| F2 (AG 2973-35 x FM 2979-01) | 24 | 59 | 32 | 1.24 |

## Claims

1. A *Brassica* plant in particular a *Brassica oleracea* plant that is highly resistant to *Thrips tabaci,* wherein highly resistant is **characterized by** the *Brassica* plant showing no symptoms and no damage, wherein the high resistance is conferred by a QTL, which is located on chromosome 2 and linked to marker BO00200 (SEQ ID NO: 1) and/or marker BO00277 (SEQ ID NO: 2) and/or marker BO00602 (SEQ ID NO: 5), and which plant is obtainable by crossing a highly resistant plant of which representative seed was deposited under NCIMB number 41760 with another cabbage plant to produce an F1 and subsequently selfing the F1 to obtain an F2 and selecting a plant therefrom that shows high resistance to *Thrips tabaci.*

2. Seed capable of growing into a plant as claimed in claim 1.

3. Progeny of a *Brassica* plant as claimed in claim 1 or progeny of plants grown from seeds as claimed in claim 2, which plant is highly resistant to *Thrips tabaci,* wherein highly resistant is **characterized by** the plant showing no symptoms and no damage, and wherein the progeny comprises the QTL, which is located on chromosome 2 and linked to marker BO00200 (SEQ ID NO: 1) and/or marker BO00277 (SEQ ID NO: 2) and/or marker BO00602 (SEQ ID NO: 5).

4. Propagation material derived from a plant as claimed in claim 1, wherein the propagation material is capable of growing into a plant is highly resistant to *Thrips tabaci,* wherein highly resistant is **characterized by** the plant showing no symptoms and no damage, and wherein the propagation material comprises the QTL, which is located on chromosome 2 and linked to marker BO00200 (SEQ ID NO: 1) and/or marker BO00277 (SEQ ID NO: 2) and/or marker BO00602 (SEQ ID NO: 5).

5. Propagation material as claimed in 4, wherein the propagation material is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, and cells.

6. Tissue culture of propagation material as claimed in claim 4 or claim 5, wherein the tissue culture comprises the QTL, which is located on chromosome 2 and linked to marker BO00200 (SEQ ID NO: 1) and/or marker BO00277 (SEQ ID NO: 2) and/or marker BO00602 (SEQ ID NO: 5).

7. Harvested part of a Brassica plant as claimed in claim 1, wherein the harvested part comprises the QTL, which is located on chromosome 2 and linked to marker BO00200 (SEQ ID NO: 1) and/or marker BO00277 (SEQ ID NO: 2) and/or marker BO00602 (SEQ ID NO: 5), which harvested part is in particular selected from the group consisting of cabbage head, curd, stem, leaf, sprout, root and seed, optionally in processed form.

8. Harvested part as claimed in claim 7, wherein the harvested part is a food product.

## Patentansprüche

1. *Brassica*-Pflanze, insbesondere eine *Brassica oleracea*-Pflanze, die hochgradig resistent gegen *Thrips tabaci* ist, wobei hochgradig resistent **dadurch gekennzeichnet ist, dass** die *Brassica*-Pflanze keine Symptome und keine Schäden zeigt, wobei die hohe Resistenz durch einen QTL verliehen wird, der auf Chromosom 2 lokalisiert und mit Marker BO00200 (SEQ ID NO: 1) und/oder Marker BO00277 (SEQ ID NO: 2) und/oder Marker BO00602 (SEQ ID NO: 5) verbunden ist, und wobei die Pflanze durch Kreuzen einer hochgradig resistenten Pflanze, von der repräsentatives Saatgut unter NCIMB-Nummer 41760 hinterlegt wurde, mit einer anderen Kohlpflanze, um eine F1 zu produzieren und anschließend Selbstung der F1, um eine F2 zu erhalten und Selektieren einer Pflanze daraus, die eine hohe Resistenz gegen *Thrips tabaci* zeigt, erhältlich ist.

2. Saatgut, das in der Lage ist, zu einer Pflanze nach Anspruch 1 heranzuwachsen.

3. Nachkommenschaft einer *Brassica*-Pflanze nach Anspruch 1 oder Nachkommenschaft von Pflanzen, die aus Saatgut nach Anspruch 2 gezüchtet wurden, wobei die Pflanze hochgradig resistent gegen *Thrips tabaci* ist, wobei hochgradig resistent **dadurch gekennzeichnet ist, dass** die Pflanze keine Symptome und keine Schäden zeigt, und wobei die Nachkommenschaft den QTL umfasst, der auf Chromosom 2 lokalisiert und mit Marker BO00200 (SEQ ID NO: 1) und/oder Marker BO00277 (SEQ ID NO: 2) und/oder Marker BO00602 (SEQ ID NO: 5) verbunden ist.

4. Vermehrungsmaterial, das von einer Pflanze nach Anspruch 1 stammt, wobei das Vermehrungsmaterial in der Lage ist, zu einer Pflanze heranzuwachsen, die hochgradig resistent gegen *Thrips tabaci* ist, wobei hochgradig resistent **dadurch gekennzeichnet ist, dass** die Pflanze keine Symptome und keine Schäden zeigt, und wobei das Vermehrungsmaterial den QTL umfasst, der auf Chromosom 2 lokalisiert und mit Marker BO00200 (SEQ ID NO: 1) und/oder Marker BO00277 (SEQ ID NO: 2) und/oder Marker BO00602 (SEQ ID NO: 5) verbunden ist.

5. Vermehrungsmaterial nach Anspruch 4, wobei das Vermehrungsmaterial aus der Gruppe ausgewählt ist, bestehend aus Mikrosporen, Pollen, Fruchtknoten, Samenanlagen, Keimlingen, Keimlingssäcken, Eizellen, Stecklingen, Wurzeln, Wurzelspitzen, Hypokotylen, Keimblättern, Stängeln, Blättern, Blüten, Antheren, Saatgut, meristematischen Zellen, Protoplasten und Zellen.

6. Gewebekultur von Vermehrungsmaterial nach Anspruch 4 oder 5, wobei die Gewebekultur den QTL umfasst, der auf Chromosom 2 lokalisiert und mit Marker BO00200 (SEQ ID NO: 1) und/oder Marker BO00277 (SEQ ID NO: 2) und/oder Marker BO00602 (SEQ ID NO: 5) verbunden ist.

7. Geernteter Teil einer Brassica-Pflanze nach Anspruch 1, wobei der geerntete Teil den QTL umfasst, der auf Chromosom 2 lokalisiert und mit Marker BO00200 (SEQ ID NO: 1) und/oder Marker BO00277 (SEQ ID NO: 2) und/oder Marker BO00602 (SEQ ID NO: 5) verbunden ist, wobei der geerntete Teil insbesondere aus der Gruppe ausgewählt ist, bestehend aus Kohlkopf, Blütenkopf, Stängel, Blatt, Spross, Wurzel und Saatgut, gegebenenfalls in verarbeiteter Form.

8. Geernteter Teil nach Anspruch 7, wobei der geerntete Teil ein Nahrungsmittelprodukt ist.

## Revendications

1. Plant de *Brassica* en particulier un plant de *Brassica oleracea* qui est hautement résistant aux *Thrips tabaci,* dans lequel hautement résistant est **caractérisé par** le plant de *Brassica* ne montrant aucun symptôme ni aucun dommage, dans lequel la haute résistance est conférée par un QTL, qui est localisé sur le chromosome 2 et lié au marqueur BO00200 (SEQ ID NO: 1) et/ou au marqueur BO00277 (SEQ ID NO: 2) et/ou au marqueur BO00602 (SEQ ID NO: 5), et ce plant pouvant être obtenu par le croisement d'un plant hautement résistant dont une semence représentative a été déposée sous le numéro NCIMB 41760 avec un autre plant de chou pour produire un F1 et l'autofécondation ultérieure du F1 pour obtenir un F2 et la sélection d'un plant provenant de celui-ci qui montre une haute résistance aux *Thrips tabaci.*

2. Semence capable de croître en un plant selon la revendication 1.

3. Descendance d'un plant de *Brassica* selon la revendication 1 ou descendance de plants mis en croissance à partir de semences selon la revendication 2, ce plant étant hautement résistant aux *Thrips tabaci,* dans laquelle hautement résistant est **caractérisé par** le plant ne montrant aucun symptôme ni aucun dommage, et dans laquelle la descendance comprend le QTL, qui est localisé sur le chromosome 2 et lié au marqueur BO00200 (SEQ ID NO: 1) et/ou au marqueur BO00277 (SEQ ID NO: 2) et/ou au marqueur BO00602 (SEQ ID NO: 5).

4. Matériau de propagation dérivé d'un plant selon la revendication 1, dans lequel le matériau de propagation est capable de croître en un plant qui est hautement résistant aux *Thrips tabaci,* dans lequel hautement résistant est **caractérisé par** le plant ne montrant aucun symptôme ni aucun dommage, et dans lequel le matériau de propagation comprend le QTL, qui est localisé sur le chromosome 2 et lié au marqueur BO00200 (SEQ ID NO: 1) et/ou au marqueur BO00277 (SEQ ID NO: 2) et/ou au marqueur BO00602 (SEQ ID NO: 5).

5. Matériau de propagation selon la revendication 4, dans lequel le matériau de propagation est sélectionné dans le groupe constitué de microspores, pollen, ovaires, ovules, embryons, sacs embryonnaires, cellules d'œuf, boutures, racines, extrémités de racines, hypocotyles, cotylédons, tiges, feuilles, fleurs, anthères, semences, cellules méristématiques, protoplastes et cellules.

6. Culture de tissu de matériau de propagation selon la revendication 4 ou la revendication 5, dans laquelle la culture de tissu comprend le QTL, qui est localisé sur le chromosome 2 et lié au marqueur BO00200 (SEQ ID NO: 1) et/ou au marqueur BO00277 (SEQ ID NO: 2) et/ou au marqueur BO00602 (SEQ ID NO: 5).

7. Partie récoltée d'un plant de Brassica selon la revendication 1, dans laquelle la partie récoltée comprend le QTL, qui est localisé sur le chromosome 2 et lié au marqueur BO00200 (SEQ ID NO: 1) et/ou au marqueur BO00277 (SEQ ID NO: 2) et/ou au marqueur BO00602 (SEQ ID NO: 5), cette partie récoltée étant en particulier sélectionnée dans le groupe constitué de tête de chou, cœur, tige, feuille, pousse, racine et semence, facultativement sous forme transformée.

8. Partie récoltée selon la revendication 7, dans laquelle la partie récoltée est un produit alimentaire.
